(19) Europäisches Patentamt
European Patent Office
Office européen des brevets

(11) Veröffentlichungsnummer: **0 108 039**
**B1**

(12) # EUROPÄISCHE PATENTSCHRIFT

(45) Veröffentlichungstag der Patentschrift: **25.01.89**

(21) Anmeldenummer: **83810440.4**

(22) Anmeldetag: **30.09.83**

(51) Int. Cl.[4]: **C 07 F 7/22,** A 01 N 55/04 // C07C27/30, C07C91/02, C07C103/54, C07D213/20, C07D215/30, C07D235/08, C07D261/02

(54) **Neue Ammonium-Stannate-(IV).**

(30) Priorität: **06.10.82 CH 5876/82**

(43) Veröffentlichungstag der Anmeldung: **09.05.84 Patentblatt 84/19**

(45) Bekanntmachung des Hinweises auf die Patenterteilung: **25.01.89 Patentblatt 89/4**

(84) Benannte Vertragsstaaten: **BE DE FR GB IT SE**

(56) Entgegenhaltungen:
**EP-A-0 048 696**
**DE-A-1 219 029**
**DE-A-2 411 016**
**US-A-3 070 615**

**Römpps Chemie-Lexikon ISBN-3440-04510-2, p. 2863**
**A.K. Sawyer "Organotin Compounds", vol. 1, pp. 84-85 + 90-93**
**S. Riethmayer "Antistatika" Gummi, Asbest, Kunststoffe, 4, 1973, pp. 298-308**

**Die Akte enthält technische Angaben, die nach dem Eingang der Anmeldung eingereicht wurden und die nicht in dieser Patentschrift enthalten sind.**

(73) Patentinhaber: **CIBA- GEIGY AG, Klybeckstrasse 141, CH- 4002 Basel (CH)**

(72) Erfinder: **Wehner, Wolfgang, Dr., Wetzbach 34, CH- 6144 Zwingenberg (CH)**
Erfinder: **Grade, Reinhardt, Dr., Tulpenweg 11, CH- 6140 Bensheim (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 108 039 B1

LIBER, STOCKHOLM 1989

**Beschreibung**

Die vorliegende Erfindung betrifft neue Ammonium-Stannat-Komplexe, welche sich durch sehr gute biocide Wirksamkeit auszeichnen.

Die Verwendbarkeit von Ammoniumsalzen, Organozinnverbindungen und auch diejenige gewisser Ammonium-Stannate als Biocide ist bekannt.

Eine allgemeine Übersicht über die Chemie der Organostannat(IV)-Komplexe gibt J. W. Nicholson in Coord. Chem. Rev., 47, 263 (1982).

In der DE-A-1 204 226 und in der DE-A-1 219 029 sind Komplexsalze beschrieben, welche durch Umsetzung von Ammoniumsalzen mit Triphenylzinnsalzen gewonnen werden. Ausserdem ist dort deren antimikrobielle Wirksamkeit beschrieben. In der EP-A-48 696 sind gewisse Ammonium-Stannate als Schädlingsbekämpfungsmittel beschrieben. Diese bekannten Verbindungen genügen jedoch nicht in jeder Hinsicht den hohen Anforderungen, welche heute an ein Biocid gestellt werden.

Die vorliegende Erfindung betrifft Verbindungen der Formel I,

$$[R^1R^2NR^3R^4]^{\oplus} [(R^7)_3 SnXY]^{\ominus} \quad (I)$$

worin $R^1$, $R^2$ und $R^3$ unabhängig voneinander $C_1$-$C_6$Alkyl oder Methylol bedeuten und $R^3$ auch Benzyl, $C_8$-$C_{22}$Alkylbenzyl, Mono- oder Dichlorbenzyl, Mono- oder Dinitrobenzyl ist, $R^4$ $C_8$-$C_{16}$Alkyl bedeutet, $R^7$ $C_3$-$C_5$Alkyl ist und X und Y unabhängig voneinander Fluor oder Chlor sind.

$R^1$, $R^2$ und $R^3$ als $C_1$-$C_6$ Alkyl sind beispielsweise geradkettige oder verzweigte Alkylreste wie Methyl, Ethyl, n-Propyl, Isopropyl, n-Butyl, sec.-Butyl, n-Amyl, Isoamyl oder n-Hexyl.

$R^3$ ist gegebenenfalls substituiertes Benzyl, wie o-, m- oder p-Methylbenzyl, 2,3-, 2,4-, 3,5- oder 2,5-Dimethylbenzyl, Nonylbenzyl, Laurylbenzyl, Tetradecylbenzyl, o-, m- oder p-Chlorbenzyl, 2,3-, 3,4-, 3,5- oder 2,5-Dichlorbenzyl, o-, m- oder p-Nitrobenzyl, 2,3-, 3,4-, 3,5- oder 2,5-Dinitrobenzyl.

$R^4$ kann als $C_8$-$C_{16}$ Alkyl n-Octyl, 2-Ethylhexyl, verzweigtes oder unverzweigtes Nonyl, Decyl, Undecyl, Dodecyl, Tridecyl, Tetradecyl oder Hexadecyl bedeuten.

$R^7$ ist als $C_3$-$C_5$ Alkyl z. B. n-Propyl, Isopropyl, sec.-Butyl, n-Amyl, bevorzugt n-Butyl.

Besonders bevorzugt wird die Verbindung N,N-Dimethyl-N-benzyl-N-n-tetradecylammonium-tri-n-butyl-dichlorostannat-(IV).

Ebenfalls bevorzugt wird die Verbindung N,N-Dimethyl-N-benzyl-N-n-tetradecylammonium-tri-n-butyl-difluorostannat-(IV).

Die Ammonium-Stannate der vorliegenden Erfindung können beispielsweise durch Umsetzung von ungefähr einem Mol eines Ammoniumsalzes der Formel II

$$[R^1R^2NR^3R^4]X \quad (II)$$

worin $R^1$, $R^2$ $R^3$, $R^4$ und X die oben angegebene Bedeutung haben mit ungefähr einem Mol eines Zinnsalzes der Formel $(R^7)_3SnY$ (III), worin $R^7$ und Y die oben angegebenen Bedeutungen haben, hergestellt werden.

Die Ammoniumsalze der Formel II und die Zinnsalze der Formel III sind in der Regel im Handel erhältliche Produkte oder können nach bekannten Methoden hergestellt werden.

Die Reaktion der Ammoniumsalze der Formel II mit dem Zinnsalz der Formel III wird vorteilhaft bei Raumtemperatur mit oder ohne Lösungsmittel (homogen oder 2-phasig), wie etwa Methanol, Ethanol, Wasser, Chloroform, Aceton, Methylenchlorid, Toluol, Xylol, etc. durchgeführt. Wird ein Lösungsmittel mitverwendet, so kann dieses nach der Reaktion verdampft werden, oder das Reaktionsprodukt wird z. B. durch Zugabe eines Ethers ausgefällt und das so erhaltene Produkt z. B. durch Umkristallisation gereinigt.

Beispiele für Ammoniumsalze der Formel II sind:

-N,N,N-Tributyl-N-tetradecylammoniumchlorid
-N,N-Dimethyl-N-n-tetradecyl-N-benzylammoniumchlorid
-N,N,N-Trimethyl-N-n-hexadecylammoniumchlorid
-N,N-Dimethyl-N-n-hexadecyl-N-benzylammoniumchlorid
-N,N,N-Trimethylol-N-n-tetradecylammoniumchlorid

Beispiele für Zinnsalze der Formel III sind:

-Tri-(n-butyl)zinnfluorid
-Tri-(n-butyl)zinnchlorid
-Tri-(n-propyl)zinnchlorid
-Tri-(n-amyl)-zinnfluorid

Die Verbindungen der Formel I zeichnen sich durch geringe Flüchtigkeit und gute Wasserlöslichkeit aus.

Mit den erfindungsgemässen Verbindungen wird ein breites Wirkungsspektrum in der Bekämpfung von tierischen und pflanzlichen Schädlingen geschaffen, woraus sich vielerlei Verwendungsmöglichkeiten, z. B. als Bactericide, Desinfektionsmittel, gegen Schleimbildung in der Papierherstellung, als Fungicide, Insekticide, Akaricide, oder Herbicide, sowie Algicide ergeben. Ausserdem eignen sich die neuen Stoffe ausgezeichnet als industrielle Antimikrobika zum Materialschutz, z. B. Schützen von Holz, Zellstoff und Papier, Textilien und

Leder, von Farben, Lacken, Antifoulingfarben und ähnlichen Beschichtungsstoffen, von optischen und anderen Gläsern, von Kühlwasser, Kunststoffen, Gummi und Klebestoffen, von Bohr- und Schneideölen, von Erdöl, Schmierstoffen, Wachsen und Treibstoffen und anderen Materialien, insbesondere auch zum Schutz biologisch abbaubarer Kunststoffe und Kunststoffzusammensetzungen, bevorzugt von weichmacherhaltigem Polyvinylchlorid oder Polyvinylidenchlorid.

Die Verbindungen werden je nach Verwendungszweck in den dem Fachmann bekannten Konzentrationsbereichen eingesetzt. Die Grenzen der gebräuchlichen Konzentrationen sind durch folgende Werte gegeben: Während in Kühlwasser bereits Konzentrationen im ppm-Bereich genügen, so sind in Antifoulingrezepturen Konzentrationen bis 40 Gew.-% üblich.

Die Verbindungen können in reiner Form oder zusammen mit Trägerstoffen als Stäube-, Streu- oder Nebelmittel appliziert werden. Sie können auch in flüssigen Streichmitteln u.ä. suspendiert werden, wobei gegebenenfalls zur Bildung von gleichmässigen Dispersionen, Netzmittel oder Emulgiermittel die gleichmässige Verteilung des Wirkstoffs fördern können. Es können weitere Biocide wie Insektizide hinzugefügt werden.

Ein bevorzugter Anwendungsbereich sind Schutzanstrichmittel, insbesondere Antifoulingfarben, auf organischer Basis, die neben den üblichen Grund- und Zusatzstoffen, 0,5 - 60 Gew.-%, vorzugsweise 3 - 25 Gew.-%, bezogen auf die Gesamtmischung, einer Verbindung der Formel I oder deren Gemische enthalten.

Übliche Grundstoffe für Antifoulingfarben sind die als Bindemittel bezeichneten und dem Fachmann bekannten Lackrohstoffe wie natürliche und synthetische Harze, homo- und copolymere Produkte mit den Monomeren Vinylchlorid, Vinylidenchlorid, Styrol, Vinyltoluol, Vinylestern, Acrylsäure und Methacrylsäure sowie deren Estern, ferner Chlorkautschuk, natürlicher und synthetischer Kautschuk, gegebenenfalls chloriert oder cyclisiert, auch Reaktionsharze wie Epoxidharze, Polyurethanharze, ungesättigte Polyester, die gegebenenfalls durch Zusatz von Härtern in filmbildende höhermolekulare Produkte überführt werden können.

Die Bindemittel können flüssig sein oder in gelöster Form vorliegen. Bei gelösten Bindemitteln, auch Thermoplasten, kann ein Schutzfilm aucn durcn verdampfen des Lösungsmittels gebildet werden. Feste Beschichtungsmittel können z. B. im Pulverbeschichtungsverfahren auf Gegenstände aufgebracht werden. Weitere übliche Grundstoffe sind z. B. Teer, Modifikatoren, Farbstoffe, anorganische oder organische Pigmente, Füllstoffe und Härter.

Es wurde ausserdem gefunden, dass Verbindungen mit der Struktur der Formel I ein für die praktischen Bedürfnisse sehr günstiges Mikrobiozid-Spektrum zum Schutze von Kulturpflanzen aufweisen. Kulturpflanzen seien im Rahmen vorliegender Erfindung beispielsweise Getreide, Mais, Reis, Gemüse, Zuckerrüben, Soja, Erdnüsse, Obstbäume, Zierpflanzen, Reben, Hopfen, Gurkengewächse (Gurken, Kürbis, Melonen), Solanaceen wie Kartoffeln, Tabak und Tomaten, sowie auch Bananen-, Kakao- und Naturkautschuk-Gewächse.

Mit den Wirkstoffen der Formel I können an Pflanzen oder Pflanzenteilen (Früchte, Blüten, Laubwerk, Stengel, Knollen, Wurzeln) dieser und verwandter Nutzkulturen die auftretenden Pilze oder Bakterien eingedämmt oder vernichtet werden, wobei auch später zuwachsende Pflanzenteile von derartigen Mikroorganismen verschont bleiben. Die Wirkstoffe sind gegen die den folgenden Klassen angehörenden phytopathogenen Pilze wirksam: Ascomycetes (z. B. Erysiphaceae, Fusarium); Basidiomycetes wie Puccinia, Fungi imperfecti (z. B. Cercospora, Septoria); Phycomycetes wie Phytophthora. Überdies wirken die Verbindungen der Formel I systemisch. Die Verbindungen der Formel I werden vorteilhaft als Beizmittel zur Behandlung von Saatgut und Vorrat (Früchte, Knollen, Körner) und Pflanzenstecklingen zum Schutz vor Pilzinfektionen sowie gegen im Erdboden auftretende phytopathogene Pilze eingesetzt.

Sie sind ausserdem gegen phytopathogene Bakterien, wie z. B. Pseudomonas sp. und Xanthomonas sp. wirksam.

Die Verbindungen der Formel I werden im Pflanzenschutz für sich allein oder zusammen mit geeigneten Trägern und/oder anderen Zuschlagstoffen verwendet. Geeignete Träger und Zuschlagstoffe können fest oder flüssig sein und entsprechen den in der Formulierungstechnik üblichen Stoffen, wie z. B. natürlichen oder regenerierten mineralischen Stoffen, Lösungs-, Dispergier-, Netz-, Haft-, Verdickungs-, Binde- oder Düngemitteln.

Der Gehalt an Wirkstoff in handelsfähigen Mitteln liegt zwischen 0,1 bis 90 %.

Zur Applikation können die Verbindungen der Formel I in den folgenden Aufarbeitungsformen vorliegen (wobei die Gewichts-Prozentangaben in Klammern vorteilhafte Mengen an Wirkstoff darstellen):

Feste Aufarbeitungsformen: Stäubemittel und Streumittel (bis zu 10 %) Granulate, Umhüllungsgranulate, Imprägnierungsgranulate und Homogengranulate, Pellets (Körner) (1 bis 80 %).

Flüssige Aufarbeitungsformen:

a) in Wasser dispergierbare Wirkstoffkonzentrate:

Spritzpulver (wettable powders) und Pasten (25 - 90 % in der Handelspackung, 0,01 bis 15 % in gebrauchsfertiger Lösung), Emulsions- und Lösungskonzentrate (10 bis 50 %; 0,01 bis 15 % in gebrauchsfertiger Lösung);

b) Lösungen (0,1 bis 20 %); Aerosole.

Die Erfindung umfasst somit ferner Mittel, die die erfindungsgemässen Verbindungen enthalten sowie die Verwendung der erfindungsgemässen Verbindungen und Mittel zur Bekämpfung von Mikroorganismen und Algen.

Die erfindungsgemäss verwendbaren Biocidgemische können auch weitere Aktivsubstanzen enthalten.

Beispiele dafür sind:

a) Organo-Schwefelverbindungen, z. B. Methylen-dithiocyanat (MBT), Isothiazolone oder 3,5-Dimethyltetrahydro-1 3,5-2H-thiadiazin-2-thion (DMTT). Solche Substanzen werden insbesondere gegen Schleimbildung bei der Papierherstellung eingesetzt.

b) Chlorierte Phenole, wie Natrium-pentachlorphenolat. Solche Verbindungen zeichnen sich durch ein breites Wirkungsspektrum aus.

c) Kupfersalze, wie Kupfersulfat sind in geringen Mengen wirksame Algicide.

d) 2,2-Dibrom-3-nitrilopropionamid (DBNPA) als Algicid, Fungicid und Bactericid.

e) Chlor und Brom sind bekannte, wirksame Algicide und Bactericide, welche besonders bei der Wasserbehandlung zum Einsatz gelangen.

f) Bei der Wasserbehandlung sind ausserdem Chlordioxid, Chlorisocyanurate und Hypochlorite gängige Biocide.

g) **Bekannte Holzbiocide**

a. Salzgemische auf Basis
- Silicofluoride
- Hydrogenfluoride
- anorg. Borverbindungen
- Chromate
- Fluoride
- Arsen (Oxid, Arsenate)
- Kupfersalze (Sulfat, Naphthenat)
- Zinn- und Zinksalze
- Quecksilberverbindungen

b. Teerölpräparate

c. Organische Wirkstoffe
- Pentachlorphenol
- Phenol
- DDT
- Dieldrin
- Lindan, Gammexan
- Chlorierte Naphthaline

h) **Bekannte Desinfektionsmittel**

a. Phenol oder Phenolderivate
b. Formaldehyd und/oder sonstige Aldehyde bzw. Derivate
c. Chlor, organ. oder anorganische Substanzen mit aktivem Chlor
d. Amphotenside.

Selbstverständlich können in solchen Formulierungen ausserdem noch weitere Substanzen und Hilfsmittel enthalten sein, wie sie üblicherweise in solchen Zubereitungen mitverwendet werden. Hierzu gehören z. B. kationische oder nichtionische oberflächenaktive Substanzen, Elektrolyte, Komplexbildner, Lösungsvermittler sowie Farb- und Duftstoffe. Diese Zusätze dienen beispielsweise zur Verbesserung des Netzvermögens, der Härtestabilität, zur Viskositätseinstellung und zur Erhöhung der Kältestabilität der Lösungen.

Die nachfolgenden Beispiele erläutern die Erfindung, ohne ihren Umfang zu begrenzen. Hierin angegebene Prozente (%) bedeuten Gewichtsprozente und Teile Gewichtsteile.

**Beispiel 1: Herstellung von N,N-Dimethyl-N-benzyl-N-tetradecylammoniumtributyldichlorstannat(IV)**

18,4 Teile N,N-Dimethy-N-benzyl-N-tetradecylammoniumchlorid werden in 80,0 Teilen Ethanol oder Chloroform gelöst. Zu dieser Lösung gibt man eine Lösung von 16,3 Teilen Tributylzinnchlorid in 100 Teilen Ethanol oder Chloroform. Nach Abziehen der flüchtigen Bestandteile verbleiben die Substanzen als viskose oder wachsähnliche Substanzen. Die $CDCl_3$-Lösungen zeigen im $^{119}Sn$-NMR-Spektrum gegenüber Tributylzinnchlorid Verschiebungen von 124,4 (Beispiel 2) ppm nach höherem Feld.

**Beispiele 2 - 8:** Analog lassen sich auch die in der folgenden Tabelle A aufgeführten Verbindungen herstellen, wobei je nach Löslichkeit der Ammonium- bzw. der Organozinnhalogenide auch noch die Löslichkeit Methanol, Ether, THF, DMF, Aceton oder Methylcellosolve in Frage kommen.

**Tabelle A**

| Bsp. | Ammonium-Kation[a)] | Stannat-Anion[a)] | Zinn NMR-Shift[b)] in $CDCl_3$ (ppm) | Eigenschaften oder Schmelzpunkt (°C) |
|---|---|---|---|---|
| 1 | N,N,N-Tri-butyl-N-tetradecylammonium | Tri-n-butyldichlorstannat | - | viskoses Öl |
| 2 | N,N-Dimethyl-N-benzyl-N-tetradecylammonium | Tri-n-butyldichlorstannat | -124,4 | viskoses Öl |
| 3 | N,N-Dimethyl-N-benzyl-N-tetradecylammonium | Tributyldifluorstannat | - | > 300 |
| 4 | N,N-Dimethyl-N-benzyl-N-tetradecylammonium | Tributylfluorchlorstannat | -46,0[c)] | viskoses Öl |
| 5 | N,N,N-Trimethyl-N-tetradecylammonium | Tributyldichlorstannat | - | viskoses Öl |
| 6 | $(CH_3)_3NC_{12}H_{25}$ | Tributyldichlorstannat | -148,8 | viskoses Öl |
| 7 | $(CH_3)_3NC_{16}H_{33}$ | Tributyldichlorstannat | -141,8 | Wachs |
| 8 | $(CH_3)_3NC_{16}H_{33}$ | Tributyldifluorstannat | - | viskoses Öl |

a) butyl = n-butyl
lauryl = n-dodecyl
tetradecyl = n-tetradecyl

b) gegen $Bu_3SnCl$ als ext. Standard
c) gegen $Bu_3SnF$ als ext. Standard

**Beispiel 9: Bestimmung der minimalen Hemmkonzentration gegen Bakterien**

Die in Caso-Pepton-Bouillon (Merck) gewachsenen üNK's (über Nacht bebrütete Kulturen) der verschiedenen Bakterienstämme:

A) Proteus vulgaris, B) Pseudomonas aeruginosa, C) Enterobacter aerogenes, D) Serratia marcencens, E) Alcaligenes denitrificans, F) Bacillus subtilis, werden jeweils in Saline 1/1000 verdünnt. Von den Suspensionen wird soviel in Caso-Pepton-Bouillon gegeben, dass die Bakterien erneut 1/1000 verdünnt werden. Danach werden jeweils die in Tabelle 1 angeführten Verbindungen in Mengen von 100 und 300 mg/l zugegeben. Nach einer Bebrütung von 24 Stunden bei 30°C im Schüttelwasserbad wird nach Trübung ausgewertet. Die minimale Hemmkonzentration (MIC) ist die Konzentration, bei der die Bouillon nicht durch Bakterienwuchs trüb wird.

Das Ergebnis ist in der folgenden Tabelle 1 veranschaulicht:

**Tabelle 1:** Bestimmung der MIC gegen Bakterien

| Verbindung aus Bsp. Nr. | Stamm A | B | C | D | E | F |
|---|---|---|---|---|---|---|
| 2 | 100 | 100 | 100 | 100 | 100 | 100 |
| 4 | 100 | 100 | 100 | 100 | 100 | 100 |
| 6 | 100 | 100 | 100 | 100 | 100 | 100 |

Aus Tabelle 1 ist die gute wuchshemmende Wirkung der Verbindungen vor allem auch gegen die schwierig zu bekämpfenden gram-negativen Bakterien zu erkennen.

**Beispiel 10: Bestimmung der minimalen Abtötungskonzentration gegen eine Bakterien-Mischkultur**

Von den in Caso-Pepton-Bouillon gewachsenen üNK's der verschiedenen Bakterienstämme

G) Escherichia coli
H) Bacillus cereus var. mycoides
I) Staphylococcus aureus
B) Pseudomonas aeruginosa
C) Enterobacter aerogenes
A) Proteus vulgaris

werden zur Herstellung der Mischkultur jeweils soviel in Tyrode gegeben, dass eine Endverdünnung von 1/1000 bzw. 1/10000 erreicht wird, und die Mischkultur wird 5 Stunden bei 30°C im Schüttelwasserbad bebrütet.

Danach werden aus den Proben 5 µl entnommen und auf Caso-Pepton-Agar getropft. Nach einer erneuten

Bebrütung bei 30°C für 24 Stunden wird visuell nach Wuchs ausgewertet.

Wie aus folgender Tabelle 2 zu erkennen ist, sind ein Teil der Verbindungen auch gegen diese schleimbildenden Bakterien hoch wirksam.

**Tabelle 2** Abtötung einer Bakterienmischkultur in Tyrode 1/1000 bzw. 1/10000 Verdünnung

| Verbindung aus Bsp. Nr. | Wuchs (mg/l) 5 1/1000 | 5 1/10000 | 10 1/1000 | 10 1/10000 | 30 1/000 | 30 1/10000 | 60 1/1000 | 60 1/10000 | 100 mg/l 1/1000 | 100 mg/l 1/10000 |
|---|---|---|---|---|---|---|---|---|---|---|
| 2 | + | + | + | + | (+) | - | (+) | - | (-) | - |
| 4 | - | - | - | - | (+) | - | (+) | - | (-) | - |
| 7 | + | (+) | + | (+) | (+) | - | - | - | - | - |

+ = Wuchs, keine Abtötung
(+) = Wuchs weniger als Kontrolle (> 10 Kolonien), geringe Abtötung
(-) = geringer Wuchs (1 - 10 Kolonien)
- = kein Wuchs, Abtötung

**Beispiel 11: Bestimmung der minimalen Hemmkonzentration gegen Pilze**

Die Untersuchung wird mit dem bekannten Agar-Inkorporations-Test in den Pilzen

K Aspergillus niger
L Aspergillus phoenicis
M Penicillium funiculosum
N Alternaria alternata
O Cladosporium cladosporioides
P Candida albicans
Q Endomyces geotrichum
R Aureobasidium pullulans
S Chaetomium globosum

im Malzextrat-Agar (Merck) durchgeführt. Zur Hemmung werden jeweils soviel an den verschiedenen Verbindungen zugegeben, dass Konzentrationen von 2, 5, 10, 50 und 100 mg/l im Agar resultieren. Die zur Hemmung des Wuchses der Pilze (von aufgetropften Pilzspuren ausgehend) benötigten Konzentrationen (mg/l) sind in Tabelle 3 veranschaulicht.

**Tabelle 3:** Bestimmung der MIC gegen Pilze (Konz. mg/l)

| Verbindung aus Bsp. Nr. | Stamm K | L | M | N | O | P | Q | R | S |
|---|---|---|---|---|---|---|---|---|---|
| 2 | 2 | 2 | 2 | 2 | 2 | 5 | 5 | 2 | 2 |
| 4 | ⩽10 | ⩽10 | ⩽10 | ⩽10 | ⩽10 | ⩽10 | ⩽10 | ⩽10 | ⩽10 |
| 6 | 2 | 2 | 5 | 2 | 2 | 5 | 50 | 2 | 2 |
| 7 | 2 | 2 | 5 | 2 | 2 | 5 | 10 | 2 | 2 |

Aus Tabelle 3 ist zu erkennen, dass die Verbindungen auch hervorragende Fungizide sind.

**Beispiel 12:** Bestimmung der Wirkung gegen Algen

a) Chlorella vulgaris

Die in Algen-Nährmedien über 14 Tage gewachsenen Kulturen von

Chlorella vulgaris

werden in Algen-Nährmedien 1/200 verdünnt. Danach werden jeweils die in Tabelle 4 angeführten Verbindungen so zugegeben, dass eine Konzentration von 3 mg/l resultiert. Nach 6 Stunden Inkubation werden jeweils 10 µl entnommen und zur Bestimmung der abtötenden Wirkung auf Algen-Agar aufgetropft. Nach einer Bebrütung von 14 Tagen unter Belichtung (14 Stunden Licht-/10 Stunden Dunkel-Wechsel) wird visuell der Wuchs im Algenmedium (Bestimmung der Wuchshemmung) und auf dem Algenagar (Bestimmung der algenabtötenden Wirkung) beurteilt.

**Tabelle 4:** Wirkung gegen Chlorella vulgaris

| Verbindung aus Bsp. Nr. | WUCHS im Medium | auf dem Agar |
|---|---|---|
| 2 | - | - |
| 4 | - | - |
| 6 | - | - |
| 7 | - | - |

- = kein Wuchs auf dem Agar, 3 ppm haben in 6 Stunden die Algen abgetötet.

b) Enteromorpha intestinalis

Die Untersuchung der Wirkung gegen die beim Meerwasser-Fouling wichtigste Grünalge Enteromorpha erfolgt in steril filtriertem Meerwasser, welches eine Erd-Schreiber-Lösung enthält. Diese Lösung setzt sich aus einem Nährstoffextrakt, Phosphat und Nitrat zusammen. Die Bebrütung von Enteromorpha intestinalis findet im Lichtthermostaten bei 18°C unter einem 14 Stunden-Licht-/10 Stunden-Dunkelwechsel statt.

Die auf diese Weise gezüchteten Algen werden den zu untersuchenden Produkten für kurze Zeit (4 Stunden) in Meerwasser ausgesetzt. Die minimale Abtötungskonzentration (MKC) wird dadurch bestimmt, dass man die Algen, nach der Einwirkungszeit einer bestimmten Menge Algicid enthaltenden Meerwassers, diesem entnimmt, sie wäscht, und nach 6 - 8-wöchiger erneuter Bebrütung in frischem Meerwasser auf Wuchs bzw. Abtötung untersucht.

Die minimale Abtötungskonzentration (MKC) gibt die Stoffmenge an, die notwendig ist, die Alge innerhalb einer bestimmten Zeit so zu schädigen, dass sie sich in frischem Meerwasser nicht mehr erholen kann und abstirbt.

Zur Bestimmung der Hemmkonzentration (MIC) befindet sich die Alge während der gesamten Versuchsdauer in Biocid enthaltenem Meerwasser (getestete Konzentration 0,5 mg/l).

**Tabelle 5:** Wirkung gegen Enteromorpha intestinalis

| Verbindung aus Bsp. Nr. | Abtötung 5 mg/l | MIC (mg/l) |
|---|---|---|
| 2 | ja | 0,5 |

Wie aus Tabellen 4 und 5 zu erkennen ist, haben die Verbindungen eine ausgeprägte algistatische und algicide Wirkung gegen Süsswasseralgen (z. B. Behandlung von Kühlwasser) und Meerwasseralgen (z. B. Bewuchsschutz für Antifouling-Farben).

**Beispiel 13:** Bestimmung der Wirkung gegen Artemia salina

Die im Handel erhältlichen Eier werden bei starker Durchlüftung zum Schlüpfen gebracht. Die 2 - 3 Tage alten Nauplien werden dann in synthetischem Meerwasser den Produkten mit verschiedener Konzentration (getestete Konzentration 2,5; 1; 0,5 und 0,25 mg/l) ausgesetzt und über einen längeren Zeitraum beobachtet.

**Tabelle 6:** Wirkung gegen Artemia salina (ca. 30 - 50 Nauplien)

| Verbindung aus | 2,5 mg/l | | | 1 mg/l | | | 0,5 mg/l | | | 0,25 mg/l | | |
|---|---|---|---|---|---|---|---|---|---|---|---|---|
| Bsp. Nr. | 6 h | 24 h | 48 h | 6 h | 24 h | 48 h | 6 h | 24 h | 48 h | 6 h | 24 h | 48 h |
| 2 | 2 - 3 | 3 | 3 | 2 | 3 | 3 | 1 - 2 | 2 - 3 | 3 | 0 - 1 | 2 | 2 - 3 |

O = wie Kontrolle
1 = geringe Veränderung
1 - 2 = Tiere angegriffen
2 = Tiere stark angegriffen
2 - 3 = Tiere fast alle tot
3 = Tiere alle tot

Aus Tabelle 6 ist die hervorragende Wirkung der Verbindungen gegen den Salinenkrebs zu erkennen. Artemia salina ist ein Verwandter der Balaniden, die die grossen Probleme bei dem Bewuchs der Schiffe verursachen.

Wegen der Wirksamkeit gegen Algen sowie gegen Krebstiere sind die Verbindungen u.a. zur Ausrüstung der Schiffsfarben geeignet.

Wegen des breiten Wirkungsspektrums, Aktivität gegen Bakterien, Algen, Pilze und Krebstiere, sind diese

Verbindungen generell für den Materialschutz, wie z. B. Dispersionsfarben, Antifouling-Farben, Wasserbehandlung, Holschutz, Bohr- und Schneideöle, Kunststoffe, Papierindustrie u.a., sowie Desinfektion verwendbar.

**Beispiel 14:** Untersuchung der Wirksamkeit in Kühlkreisläufen

Die im Freien stehenden (natürliche Sonneneinstrahlung, Staubeinfall, Witterungseinflüsse) Kühlkreisläufe bestehen aus:

a) einem Kunststoffass mit einem Volumen von 113 l und einem Überlauf,
b) einer Pumpe (21 l/min. bei 3 m Förderhöhe),
c) einem Kühlturm mit Oregon-(Splint), Oregon-(Kernholz), Eiche-, Ficht-, Asbestzement- und PVC-Platte.

Die Frischwasser-Zufuhr wird so eingestellt, dass der Spritz- und Verdunstungsverlust ausgeglichen wird und die Biocide in 24 Stunden ungefähr 1 : 2 verdünnt werden.

Die Kühlkreisläufe sind durch den natürlichen Staubeinfluss und nicht durch gezielte Beimpfung infiziert.

Zur Verhinderung der Schleimbildung und des Algenwuchses wird der Kühlkreislauf mit

100 ppm/Woche für 3 Monate und 50 ppm/Woche für weitere 2 Monate mit folgender Formulierung behandelt:

4 % der Verbindung aus Beispiel 2
15 % N,N-Dimethyl-N-benzyl-N-tetradecylammoniumchlorid
8 % Isopropanol
73 % $H_2O$

Ausgewertet wird der Versuch durch visuelle Beobachtung des Bewuchses auf den Platten.

Der unbehandelte Kreislauf zeigt schon nach 4 Wochen den ersten Algenbewuchs, die Hölzer sind nach 5 Monaten verschleimt und mit einer dicken Algenschicht überzogen. Der mit 100 ppm/Woche für die ersten drei Monate und mit 50 ppm für die nächsten zwei Monate behandelte Kreislauf zeigt keinerlei Anzeichen von beginnender Schleimbildung und Algenbewuchs.

**Beispiel 15:** Wachstum auf Holz

Holzklötzchen aus Fichte, 7 x 10 x 10 $mm^3$ gross, werden 30 Minuten im Vakuum getrocknet. Dann werden die Klötzchen vakuumimprägniert, indem sie in 100 ml dest. Wasser + Bioxid für 30 Minuten im Vakuum belassen werden und danach für 18 Stunden im Wasser einer Druckbehandlung (2 atü durch Pressluft) ausgesetzt werden. Die so behandelten Holzstücke werden erst einer Auslaugung im fliessenden Wasser unterworfen und dann getrocknet.

Die getrockneten Holzstückchen werden auf Kartoffel-Glucose-Agar gelegt und das Holz sowie der umliegende Agar mit 0,1 ml einer Sporensuspension vom Aureobasidium pullulans angeimpft. Nach 4-wöchiger Bebrütung bei 28°C wird der Wuchs nach folgendem Schema ausgewertet:

1 = Wuchs auf dem Holz, wie unbehandelte Probe
2 = Wuchs auf dem Holz, schwächer als Kontrolle
3 = kein Wuchs auf dem Holz
4 = kein Wuchs auf dem Holz, Hemmzone

| Verbindung aus Bsp. Nr. | Konz. % in $H_2O$ | ungewässert | 5 Tage ausgelaugt |
|---|---|---|---|
| 2 | 0,05 | 4 | 4 |
| | 0,01 | 4 | 4 |

Aus der Tabelle ist die hervorragende Wirkung der Verbindungen im Holzschutz - auch noch nach Wasserlagerung - zu erkennen.

## Patentansprüche

1. Verbindungen der Formel I,

$$[R^1R^2NR^3R^4]^{\oplus} [(R^7)_3 SnXY]^{\ominus} \qquad (I)$$

worin $R^1$, $R^2$ und $R^3$ unabhängig voneinander $C_1$-$C_6$Alkyl oder Methylol bedeuten und $R^3$ auch Benzyl, $C_8$-

$C_{22}$Alkylbenzyl, Mono- oder Dichlorbenzyl, Mono- oder Dinitrobenzyl ist, $R^4$ $C_8$-$C_{16}$Alkyl bedeutet, $R^7$ $C_3$-$C_5$Alkyl ist und X und Y unabhängig voneinander Fluor oder Chlor sind.

2. Verbindungen gemäss Anspruch 1, worin $R^7$ n-Butyl ist.

3. Die Verbindung N,N-Dimethyl-N-benzyl-N-n-tetradecylammoniumtri-n-butyl-dichlorostannat-(IV) gemäss Anspruch 1.

4. Die Verbindung N,N-Dimethyl-N-benzyl-N-n-tetradecylammoniumtri-n-butyl-difluorostannat-(IV) gemäss Anspruch 1.

5. Zusammensetzung enthaltend mindestens eine Verbindung gemäss Anspruch 1 und einen inerten Trägerstoff.

6. Verwendung von Verbindungen gemäss Anspruch 1 zur Bekämpfung von tierischen und pflanzlichen Schädlingen.

## Claims

1. A compound of the formula I

$$[R^1R^2NR^3R^4]^{\oplus} [(R^7)_3 SnXY]^{\ominus} \quad (I)$$

in which $R^1$, $R^2$ and $R^3$ independently of one another are $C_1$-$C_6$alkyl or methylol and $R^3$ is also benzyl, $C_8$-$C_{22}$alkylbenzyl, monochlorobenzyl dichlorobenzyl, mononitrobenzyl or dinitrobenzyl, $R^4$ is $C_8$-$C_{16}$alkyl, $R^7$ is $C_3$-$C_5$alkyl and X and Y independently of one another are fluorine or chlorine.

2. A compound according to Claim 1, in which $R^7$ is n-butyl.

3. The compound N,N-dimethyl-N-benzyl-N-n-tetradecylammonium tri-n-butyldichlorostannate-(IV) according to Claim 1.

4. The compound N,N-dimethyl-N-benzyl-N-n-tetradecylammonium tri-n-butyldifluorostannate-(IV) according to Claim 1.

5. A composition containing at least one compound according to Claim 1 and an inert carrier.

6. The use of a compound according to Claim 1 for controlling animal and plant pests.

## Revendications

1. Composés de formule I

$$[R^1R^2NR^3R^4]+ [(R^7)3 SnXY]^6 \quad (I)$$

dans laquelle $R^1$, $R^2$ et $R^3$, indépendamment les uns des autres, sont chacun un radical alkyle en $C_1$-$C_6$ ou le radical méthylol, et $R^3$ est aussi le radical benzyle, un radical (alkyle en $C_8$-$C_{22}$)benzyle, ou encore le radical mono- ou dichlorobenzyle, ou mono- ou dinitrobenzyle, $R_4$ est un radical alkyle en $C_8$-$C_{16}$, $R^7$ est un radical alkyle en $C_3$-$C_5$, et X et Y, indépendamment l'un de l'autre, sont chacun le fluor ou le chlore.

2. Composés selon la revendication 1, dans lesquels $R^7$ est le radical n-butyle.

3. Le composé tri-n-butyl-dichlorostannate-(IV) de N,N-diméthyl-N-benzyl-N-n-tétradécylammonium selon la revendication 1.

4. Le compose tri-n-butyl-difluorostannate-(IV) de N,N-diméthyl-N-benzyl-N-n-tétradécylammonium selon la revendication 1.

5. Composition contenant au moins un composé selon la revendication 1 et un support inerte.

6. Utilisation de composés selon la revendication 1, pour la maitrise dés parasites animaux et végétaux.